# EUROPEAN PATENT APPLICATION

(11) **EP 2 522 340 A1**
(43) Date of publication of application: **14.11.2012**
(21) Application number: 12176366.8
(22) Date of filing: 18.06.2007
(51) Int. Cl.: A61K 9/72, A61K 31/137, A61K 31/427, A61K 31/56, A61P 11/06, A61P 11/08, A61K 9/00

(54) **Method of using a thiazole derivative**

(30) Priority: 19.06.2006 US 814545 P
(62) Divisional of application: 07767448.9
(71) Applicant: OTSUKA PHARMACEUTICAL CO., LTD., Chiyoda-ku Tokyo 101 (JP)
(72) Inventor: Molfino, Nestor A., Rockville, MD Maryland 20850 (US); Saito, Kosuke, Princeton, NJ New Jersey 08540 (US); Nagamoto, Hisashi, Tokushima, 771-0192 (JP)
(74) Representative: HOFFMANN EITLE

(57) **Abstract**

This invention relates to a combination of tetomilast and at least one beta₂-adrenergic receptor agonist for use in the treatment of a disease, disorder, or condition. The invention further relates to the combination of an anti-inflammatory steroid, tetomilast and at least one beta₂-adrenergic receptor agonist for use in the treatment of a disease, disorder, or condition.

## Description

### Technical Field

The present invention relates to methods of using a thiazole derivative, in particular, tetomilast, in combination with a beta₂-adrenergic receptor agonist and/or an anti-inflammatory steroid.

### Background Art

Tetomilast (2-(3,4-diethoxyphenyl)-4-(2-carboxy-6-pyridyl)thiazole; 6-[2-(3,4-diethoxyphenyl)-1,3-thiazol-4-yl]pyridine-2-carboxylic acid) is a thiazole derivative useful for the treatment of a variety of diseases. The chemical structure of tetomilast is provided in Formula 1 (see also http://www.who.int/druginformation/vol18num2_2004/propl ist91.pdf).

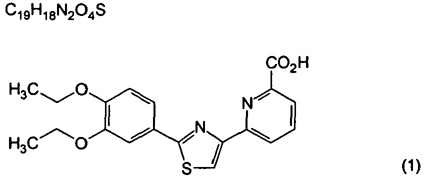

Tetomilast has been implicated in several specific functions in activated neutrophils both in vitro and in vivo, including superoxide production (e.g., U.S. Patent No. 5,643,932 (RE37,556), fully incorporated by reference) and adhesion to endothelium (e.g., U.S. Patent No. 6,291,487, fully incorporated by reference). Tetomilast has also been shown to improve airway resistance and peak expiration flow in guinea pig models of pulmonary disease (e.g., U.S. Patent Publication No. 2004/0147563, fully incorporated by reference). Furthermore, tetomilast pretreatment resulted in dose-related suppression of lipopolysaccharide (LPS)-induced cytokine production (tumor necrosis factor alpha [TNF-α], interleukin 1-beta [IL-1β], and interleukin-6 [IL-6]) in human whole blood from normal volunteers in vitro (e.g. U.S. Patent No. 6,291,487), implying a possible inhibitory effect on activated monocytes.

Tetomilast has been suggested for the treatment of chronic obstructive pulmonary disease (COPD) and a number of other diseases, disorders, or conditions (e.g., U.S. Patent Publication No. 2004/0147563) but its interaction with other drugs has not been previously analyzed. Therefore, tetomilast has not been suggested for use or used in combination with other drugs to treat, for example, respiratory diseases, disorders, or conditions.

A respiratory disease, disorder, or condition is characterized by the inflammation of airways which results in either airway restriction (i.e., reduction in the functional volume of the lungs) or airway obstruction (i.e., impediment to the rate of flow into and out of the lungs). Respiratory disease is the number three killer in America and is the primary cause of death for children under one year old.

COPD is characterized by the progressive development of airflow limitation (airway obstruction) (Pauwels R. A., et al.: Am. J. Respir. Crit. Care Med., 2001 (163), 1256-1276). COPD is one of the major causes of chronic morbidity and mortality throughout the world, and in the Asia-Pacific region, it is foreseen that patients with COPD will rapidly increase within the next 20 years due to an increase in the aging population and in the number of smokers.

Conventional treatments for respiratory disease are designed to mitigate airway inflammation, thus alleviating airway restriction. One such method known in the art involves administering anti-inflammatory agents, such as corticosteroids, which have a potent inhibitory effect on the production of cytokines. However, most large-scale clinical studies have shown that anti-inflammatory steroids cannot improve the long-term progressive decline of pulmonary function in patients with COPD, limiting their usefulness to only temporary relief of the symptoms of respiratory disease (Pauwels R. A., et al.: N. Engl. J. Med., 1999 (340), 1948-1953; Vestbo J. et al.: Lancet, 1999 (352), 1819-1823; Burge P. S., et al.: BMJ, 2000 (320), 1297-1303).

A second method known in the art for treating airway inflammation is to elevate intracellular levels of cyclic adenosine 3',5'-monophosphate (cAMP). In respiratory disease, the principal cells involved in the associated bronchoconstriction and inflammatory process are subject to inhibitory control by cAMP. Hence, compounds that raise levels of cAMP are often administered to treat airway inflammation and airway restriction associated with respiratory disease (Rabe K. F. et al.: Eur Respir J, 1995 (8), 637-342).

One mechanism known to elevate CAMP levels in respiratory cells is to administer bronchodilatory drugs such as beta₂-adrenergic receptor agonists, and the like. These drugs activate receptors on airway smooth muscle. Once activated, these receptors stimulate adenylate cyclase to synthesize cAMP. Thus, beta₂-adrenergic receptor agonists, such as albuterol (World Health Organization recommended name, salbutamol) and salmeterol, are conventional treatments for respiratory disease. However, like anti-inflammatory steroids, these bronchodilatory drugs also cannot improve the long-term deterioration of pulmonary function in patients with respiratory disease, limiting their usefulness to only temporary relief of the symptoms of these diseases (Billah M. M. et al.: JPET, 2002 (302), 127-137).

A second mechanism known for elevating cAMP levels in respiratory cells is to inhibit cAMP breakdown by blocking cyclic nucleotide phosphodiesterases (PDEs). There are at least eleven PDE enzyme families that degrade cAMP and/or cGMP. Among the forms of PDE is the cAMP-specific isozyme PDE4, which is a major component in airway smooth muscle and in inflammatory and immunocompetent cells. However, it has been reported that these drugs have adverse side effects as well, for example, nausea, vomiting and increasing the secretion of acid in the stomach
(Barnes P. J.: N. Engl. J. Med., 2000 (343) No. 4, 269-280).

### Disclosure of Invention

The instant invention relates to a method of treating a patient comprising administering to a patient a therapeutically effective amount of tetomilast. In an embodiment of the invention, at least one beta₂-adrenergic receptor agonist may be administered with tetomilast. In another embodiment of the invention, an anti-inflammatory steroid may also be administered with tetomilast and at least one beta₂-adrenergic receptor agonist. Tetomilast, the beta₂-adrenergic receptor agonist, and/or the anti-inflammatory steroid may be administered simultaneously, separately, or sequentially. The instant invention may be used to treat or prevent a respiratory disease, disorder, or condition, such as chronic obstructive pulmonary disease (COPD).

It is to be understood that both the foregoing general description and the following detailed description are exemplary and explanatory only and are not restrictive of the invention, as claimed.

The accompanying drawings, which are incorporated in and constitute a part of this specification, illustrate several embodiments of the invention and together with the description, serve to explain the principles of the invention.

### Brief Description of Drawings

Figure 1 is a graph showing the mean changes in trough FEV₁ from baseline (day 1 predose) for the tetomilast and placebo groups.

Figure 2 is a graph showing the mean changes in peak FEV₁ from baseline (day 1 predose) for the tetomilast and placebo groups.

Figure 3 is a graph showing the mean changes in FEV₁ for the tetomilast and placebo groups.

### Best Mode for Carrying Out the Invention

The methods contemplated by the instant invention comprise administering to a patient a therapeutically effective amount of tetomilast, a thiazole derivative described in U.S. Patent Publication No. 2004/0147563, which is fully incorporated herein by reference. Embodiments of the method further comprise administering at least one beta₂-adrenergic receptor agonist, and optionally at least one anti-inflammatory steroid.

The phrase "a therapeutically effective amount," as used herein, refers to an amount of active compound sufficient to stimulate therapeutic physiological and pharmacological responses in a patient. Preferably, the effective amount of the active compound stimulates the desired physiological and pharmacological responses without producing adverse effects.

A patient is hereby defined as any person or non-human animal in need of tetomilast or its combinations, or any subject for whom treatment with tetomilast or its combinations may be beneficial, including humans and non-human animals. Such non-human animals to be treated include all domesticated and feral vertebrates. The patient may have a steady-state plasma level of any one of the active compounds of the invention at the time of treatment.

The terms "treatment" and "treating" as used herein refer to both prophylactic and symptomatic modes of therapy.

The method of the instant invention may be used to prevent or to treat a disease, disorder, or condition, such as a respiratory disease, disorder, or condition. Respiratory diseases, disorders, or conditions include allergic and inflammatory diseases of the lungs and upper respiratory tract. Exemplary respiratory diseases, disorders, or conditions include, but are not limited to, asthmatic conditions (e.g., allergic asthma, bronchial asthma, exercise-induced asthma, pollution-induced asthma, and cold-air induced asthma), acute respiratory distress syndrome, chronic or acute bronchoconstriction, chronic obstructive pulmonary disease (COPD), bronchitis (e.g., acute bronchitis, chronic bronchitis, obstructive bronchitis, spastic bronchitis, allergic bronchitis), emphysema, pneumoconiosis, small airway obstruction, sinusitis, bronchiectasis, and rhinitis (e.g., seasonal or perennial rhinitis).

An embodiments of the invention includes a method of preventing or treating chronic obstructive pulmonary disease (COPD). COPD is often associated with the progressive development of airflow limitation (airway obstruction), wherein the inflammatory cells found in the bronchoalveolar lavage fluid and sputum of patients are neutrophils.

In addition to the various diseases, disorders, or conditions disclosed in U.S. Patent No. 5,643,932, U.S. Patent No. 6,291,487, and U.S. Patent Publication No. 2004/14563, other exemplary diseases, disorders, or conditions that may be prevented or treated by tetomilast include, but are not limited to, restenosis, gynecological disorder, osteoarthritis, eczema, lupus nephritis, allograft nephropathy, congenital obstructive nephropathy, celiac disease, Graves' disease, thyroiditis, muscular dystrophy, spinal muscular atrophy, demyelinating disease of the central nervous system (CNS), encephalomyelitis, myositis, and Graft-versus-host disease (GvHD).

Tetomilast

Tetomilast (2-(3,4-diethoxyphenyl)-4-(2-carboxy-6-pyridyl)thiazole; 6-[2-(3,9-diethoxyphenyl)-1,3-thiazol-4-yl]pyridine-2-carboxylic acid) is a thiazole derivative useful for the treatment of a variety of diseases and described in U.S. Patent Publication No. 2004/0147563. The chemical structure of tetomilast is provided in Formula 1.

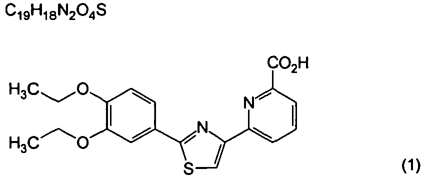

The tetomilast as used herein and represented by Formula 1 of the instant invention includes its prodrugs, conjugates, or any other form that will provide an active form of tetomilast. The tetomilast as used herein is further intended to include its salt forms (both acidic and basic), solvates, polymorphs, etc.

The Beta₂-Adrenergic Receptor Agonist

The phrase "beta₂-adrenergic receptor agonist," as used herein, refers to compounds which stimulate the beta₂-adrenergic receptor. Beta₂-adrenergic receptors are the predominant receptors in bronchial smooth muscle. Once activated, the beta₂-adrenergic receptor stimulates adenylate cyclase to synthesize cAMP. Equivalent phrases used in this application and in the art include: "beta₂-agonist" and "β₂ agonist." The beta₂-adrenergic receptor agonist, as used herein, includes its racemates, stereoisomers and their mixtures, prodrugs, conjugates, or any other form that will provide an active form of the beta₂-adrenergic receptor agonist. Furthermore, any of the β₂-adrenergic receptor agonists as used herein is intended to include its salt forms (both acidic or basic), solvates, polymorphs, etc.

Beta₂-adrenergic receptor agonists include, for example, but are not limited to: albuterol (salbutamol), AR-C68397AA, arformoterol, bambuterol, bitolterol, broxaterol, carbuterol, CHF-1035, clenbuterol, dopexamine, fenoterol, formoterol, hexoprenaline, HOKU-81, ibuterol, isoetharine, isoprenaline, KUL-1248, levosalbutamol, mabuterol, meluadrine, metaproterenol, nolomirole, orciprenaline, pirbuterol, procaterol, reproterol, rimiterol, ritodrine, salmefamol, salmeterol, sibenadet, soterenot, sulfonterol, TA-2005, terbutaline, tiaramide, tulobuterol, epinephrine, norepinephrine, colterol, ethyinorepinephrine, isoproterenol, metaproterenol, ephedrine, GSK-597901, GSK-159797, GSK-678007, GSK-642444, GSK-159802, (-)-2-[7(S)-[2(R)-Hydroxy-2-(4-hydroxyphenyl)ethylamino]-5,6,7,8-terahydro-2-naphthyloxy]-N,N-dimethylacetamide hydrochloride monohydrate, carmoterol, QAB-149 and 5-[2-(5,6-diethylindan-2-ylamino)-1-hydroxyethyl]-8-hydroxy-1H-quinolin-2-one, 4-hydroxy-7-[2-{[2-{[3-(2-phenylethoxy)propyl]sulfonyl}ethyl]amino}ethyl]-2 (3H)-benzothiazolone, 1-(2-fluoro-4-hydroxyphenyl)-2-[4-(1-benzimidazolyl)-2-methyl-2-butylamino]ethanol, 1-[3-(4-methoxybenzylamino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hyrdoxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-one, 1-(4-amino-3-chloro-5-trifluoromethylphenyl)-2-tertbutylamino)ethanol, and 1-(4-ethoxycarbonylamino-3-cyano-5-fluorophenyl)-2-(tert-butylamino)ethanol.

Beta₂-adrenergic receptor agonists may be classified as non-selective and selective beta₂-agonists. Non-selective beta₂-agonists refer to those compounds which act upon all classes of α- and β-adrenergic receptors and include, but are not limited to, epinephrine, norepinephrine, colterol, ethyinorepinephrine, isoproterenol, metaproterenol, and ephedrine. Selective beta₂-agonists refer to those compounds which specifically act upon P₂-adrenergic receptors and include, but are not limied to, albuterol (salbutamol), AR-C68397AA, arformoterol, bambuterol, bitolterol, broxaterol, carbuterol, CHF-1035, clenbuterol, dopexamine, fenoterol, formoterol, hexoprenaline, HOKU-81, ibuterol, isoetharine, isoprenaline, KUL-1248, levosalbutamol, mabuterol, meluadrine, metaproterenol, nolomirole, orciprenaline, pirbuterol, procaterol, reproterol, rimiterol, ritodrine, salmefamol, salmeterol, sibenadet, soterenot, sulfonterol, TA-2005, terbutaline, tiaramide, tulobuterol, GSK-597901, GSK-159797, GSK-678007, GSK-642444, GSK-159802, (-)-2-[7(S)-[2(R)-Hydroxy-2-(4-hydroxyphenyl)ethylamino]-5, 6,7,8-terahydro-2-naphthyloxy]-N,N-dimethylacetamide hydrochloride monohydrate, carmoterol, QAB-149 and 5-[2-(5,6-diethylindan-2-ylamino)-1-hydroxyethyl)-8-hydroxy-1H-quinolin-2-one, 4-hydroxy-7-[2-{[2-{[3-(2-phenylethoxy)propyl]sulfonyl}ethyl]amino}ethyl]-2 (3H)-benzothiazolone, 1-(2-fluoro-4-hydroxyphenyl)-2-[4-(1-benzimidazolyl)-2-methyl-2-butylamino]ethanol, 1-[3-(4-methoxybenzylamino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hyrdoxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-one, 1-(4-amino-3-chloro-5-trifluoromethylphenyl)-2-tertbutylamino)ethanol, and 1-(4-ethoxycarbonylamino-3-cyano-5-fluorophenyl)-2-(tert-butylamino)ethanol.

Beta₂-adrenergic receptor agonists may also be classified as long-acting or short-acting. "Long-acting" refers to a drug which has an effect on the bronchi for about six hours or more, and may last up to about twelve hours in some instances. "Short-acting" refers to a drug which has an effect on the bronchi for less than about six hours.

Specific embodiments of beta₂-adrenergic receptor agonists contemplated by this invention include albuterol or salmeterol. In a further embodiment, the beta₂-adrenergic receptor agonist is salmeterol xinafoate.

The Anti-Inflammatory Steroid

The phrase "anti-inflammatory steroid," as used herein, is well known in the art and refers to compounds that reduce or prevent inflammation, and includes its stereoisomers and their mixtures, esters, prodrugs, conjugates, or any other form that will provide anti-inflammatory activity. The anti-inflammatory steroid as used herein is also intended to include its salts (both acidic and basic), solvates, polymorphs, etc.

Examples of anti-inflammatory steroids include, but are not limited to, corticosteroids, alcolmetasone dipropionate, beclomethasone, budesonide, butixocort propionate, ciclesonide, clocortolone pivalate, deflazacort, dexamethasone, dexamethasone palmitoate, dexamethasone sodium phosphate, deprodone propionate, fimexolone, fluocinolone acetonide, fluocinonide, flunisolide, fluticasone, fluticasone propionate, halobetasol propionate, halopredone acetate, halometasone, hydrocortisone, hydrocortisone aceponate, hydrocortisone acetate, hydrocortisone sodium succinate, hydrocortisone probutate, loteprednol etabonate, methylprednisolone, methylprednisolone aceponate, methylprednisolone suleptanate, mometasone, naflocort, prednisone, prednisolone, prednisolone farnesylate, prednisolone sodium phosphate, prednicarbate, rimexolone, rofleponide, triamcinolone (e.g., as the acetonide), tipredane, 6α,9α-difluoro-17α-[(2-furanylcarbonyl)oxy]-11β-hydroxy-16α-methyl-3-oxo-androsta-1,4-diene-17b-carbothioic acid S-fluoromethyl ester, 6α,9α-difluoro-11β-hydroxy-16α-methyl-3-oco-17α-propionyloxy-androsta-1,4-diene-17β-carbothioic acid S-(2-oxo-tetrahydro-furan-3S-yl) ester, RPR-106541, and 5T-126 (SSP-Torii).

Methyl prednisolone and prednisone are oral and injectable forms of anti-inflammatory corticosteroids; they are available from numerous branded and generic pharmaceutical companies. Beclomethasone dipropionate is sold as an aerosol for inhalation under the names Beconase^{®} and Beconase AQ^{®} by GlaxoSmithKline. Fluticasone propionate is sold under the name Flonase^{®} by GlaxoSmithKline. Triamcinolone acetonide is sold by Rhone-Poulenc Roher under the name Nasocort^{®} as a nasal spray and aerosol. Flunisolide is sold as a nasal solution under the name Nasalide^{®} and Nasarel^{™} by Roche Laboratories. Dexamethasone is sold as the sodium phospate salt by Medeva Pharmaceuticals, Inc. under the name Dexacort^{™} Phosphate. Mometasone furoate is sold as the monohydrate as a nasal preparation by Schering Corp under the name Nasonex^{®}. Budesonide is yet another inhaled corticosteroid used in treating pulmonary diseases. Budesonide is marketed by Astra Pharmaceuticals, L.P. as a powder in a Turbuhaler^{®} device under the name Pulmicort Turbuhaler^{®}. All of these drugs and nasal preparations or oral or injectable formulations can be found in the 1999 edition of the Physicians' Desk Reference® (PDR), published by Medical Economics Corporation, Inc, of New Jersey, USA.

A specific embodiment of anti-inflammatory steroids contemplated by this invention includes fluticasone. In a further embodiment of the invention, the anti-inflammatory steroid is fluticasone propionate.

Preparation of Active Compounds

As discussed above, the active compounds of the instant invention may also be present, for example, in the form of their salts and/or as solvates (e.g., hydrates), and/or in the form of their N-oxides, etc.

The salt form of the active compound includes its base salts and acid addition salts. In an embodiment of the invention, the salt may be a "pharmaceutically acceptable salt," which may confer on the active compound improved pharmacokinetic properties as compared to the free form of the active compound. The pharmaceutically acceptable salt form of the active compound may also initially confer a desirable pharmacokinetic property on the active compound which it did not previously possess, and may even positively affect the pharmacodynamics of the active compound with respect to its therapeutic activity in the body.

The pharmacokinetic properties of the active compound which may be favorably affected include, for example, the manner in which the active compound is transported across the cell membrane, which in turn may directly and positively affect the absorption, distribution, biotransformation and excretion of the active compound.

The salt forms of the active compounds of the instant invention may be prepared by conventional means. Where the active compound contains an acid group, a suitable salt thereof may be formed by reacting the compound with a sufficient amount of an appropriate base to provide the corresponding base addition salt. Examples of such bases are alkali metal hydroxides including potassium hydroxide, sodium hydroxide, and lithium hydroxide; alkaline earth metal hydroxides such as barium hydroxide and calcium hydroxide; alkali metal alkoxides (e.g., potassium ethanolate and sodium propanolate); and various organic bases such as piperidine, diethanolamine, and N-methylglutamine. Also included are the aluminum salts of the active compounds of the instant invention.

Accordingly, the pharmaceutically acceptable base salts of the active compounds include, but are not limited to: aluminum, ammonium, calcium, copper, ferric, ferrous, lithium, magnesium, manganic, manganous, potassium, sodium, and zinc salts.

Alternatively, acid addition salts may be formed by treating a free base form of the active compounds with a sufficient amount of a pharmaceutically acceptable organic or inorganic acid. Examples include, but are not limited to, hydrohalides such as hydrochloride, hydrobromide, hydroiodide; other mineral acids and their corresponding salts such as sulfate, nitrate, phosphate, etc.; alkyl- and monoarylsulfonates such as ethanesulfonate, toluene sulfonate, and benzenesulfonate; and other organic acids and their corresponding salts such as acetate, tartrate, maleate, succinate, citrate, benzoate, salicylate, ascorbate, etc.

Accordingly, the pharmaceutically acceptable acid addition salts of the active compounds of the instant invention include, but are not limited to: acetate, adipate, alginate, arginate, aspartate, benzoate, benzenesulfonate (besylate), bisulfate, bisulfite, bromide, butyrate, camphorcitrate, cyclopentanepropionate, digluconate, dihydrogenphosphate, dinitrobenzoate, dodecylsulfate, ethanesulfonate, fumarate, galacterate (from mucic acid), galacturonate, glucoheptanoate, gluconate, glutamate, glycerophosphate, hemisuccinate, hemisulfate, heptanoate, hexanoate, hippurate, hydrochloride, hydrobromide, hydroiodide, 2-hydroxyethanesulfonate, iodide, isethionate, iso-butyrate, lactate, lactobionate, malate, maleate, malonate, mandelate, metaphosphate, methanesulfonate, methylbenzoate, monohydrogenphosphate, 2-naphalenesulfonate, nicotinate, nitrate, oxalate, oleate, pamoate, pectinate, persulfate, phenylacetate, 3-phenylpropionate, phosphate, phosphonate, and phthalate.

As discussed above, the active compounds of the instant invention also include their stereoisomers. The term "stereoisomer" refers to compounds that have the same molecular formula and that have their atoms joined in the same order but differ in the way their atoms are arranged in space. Stereoisomers include enantiomers (also called optical isomers, which are stereoisomers that are mirror images and have equal but opposite specific rotation) and diastereomers (stereoiosomers that are not mirror images).

The active compounds of the instant invention also include their esters. The term "ester" refers to an active compound made from an esterification reaction in which a carboxylic acid reacts with an alcohol through the loss of a molecule of water.

Pharmaceutical Compositions, Formulations and Methods of Administration

The pharmaceutical compositions of the instant invention comprise any one or more of the active compounds described above. The pharmaceutical composition may also comprise a pharmaceutically acceptable carrier in accordance with the properties and expected performance of such carriers for administration to a patient.

As used herein the term "carrier" includes acceptable diluents, excipients, adjuvants, vehicles, solubilization aids, viscosity modifiers, preservatives and other agents known for providing favorable properties in the final pharmaceutical composition to be administered to the patient.

More particularly, the carriers contemplated by the instant invention include: acidifying and alkalizing agents to obtain a desired or predetermined pH; antimicrobial agents (including antibacterial, antifungal, and antiprotozoal agents); antioxidants to protect the ingredients of the pharmaceutical composition from damage or degradation; buffering agents to maintain a desired pH; chelating agents to maintain the ionic strength; dispersing and suspending agents; emulsifying agents; excipients; preservatives; stabilizers; sugars; and surfactants. Therapeutically acceptable examples of these various classes of carriers will be known to those of skill in the art and specific examples are discussed below.

In an embodiment of the invention, the method comprises administering one of the following combinations: 1) tetomilast and albuterol; 2) tetomilast and salmeterol xinafoate; 3) tetomilast, albuterol, and fluticasone propionate; and 4) tetomilast, salmeterol xinafoate, and fluticasone propionate.

As used herein, combinations of active compounds may be "more efficacious" if the combinations potentiate the physiological and pharmacological responses when compared to the active compound administered alone. Alternatively, the combinations of active compounds may be "more efficacious" if the combination produces the same level of physiological and pharmacological responses as the active compounds administered alone, and the dose of each active compound in the combination is lower than the dose required to produce the same level of response when the active compound is administered alone. Finally, the combinations of active compounds may be "more efficacious" if the combination produces additional physiological and pharmacological responses when compared to the active compounds administered alone.

The active compounds of the instant invention may be administered by any suitable route, depending on the nature of the disease, disorder, or condition to be treated. For example, the compounds may be administered orally as syrups, tablets, capsules, or lozenges, etc., either as controlled-release preparations or immediate release preparations. Alternatively, the compounds of the instant invention may be administered by inhalation as a dry powder, a solution, a dispersion, etc. The compounds may also be administered topically as creams, ointments, lotions, nasal sprays, aerosols, etc. Finally, the compounds may alternatively be used for parenteral, intradermal, subcutaneous, intramuscular, or intravenous injection.

The pharmaceutical formulations may be prepared by any of the methods known in the pharmaceutical arts. In general, the formulations may be prepared by uniformly and intimately bringing into association the active compound(s) with liquid carriers or finely divided solid carriers or both and then, if necessary, shaping the product into the desired formulation.

Formulations of the present invention suitable for oral administration may be presented as discrete units such as capsules, cachets or tablets containing a predetermined amount of the active compound(s). The formulation may be a powder or granules; a solution or suspension in an aqueous or a non-aqueous liquid; or an oil-in-water liquid emulsion or a water-in-oil liquid emulsion. The active compound(s) may also be presented as a bolus, electuary or paste.

Oral compositions generally include an inert diluent or an edible carrier. The active compound(s) may be incorporated with excipients. Oral compositions may also be prepared using a fluid carrier for use as a mouthwash, wherein the active compound in the fluid carrier is applied orally and swished and expectorated or swallowed. Pharmaceutically compatible binding agents, and/or adjuvant materials can be included as part of the composition. Binding agents may include, for example, microcrystalline cellulose, gum tragacanth, or gelatin. Excipients may include, for example, starch or lactose. Disintegrating agents such as alginic acid, or corn starch may also be added. Lubricants, such as magnesium stearate, or glidants such as colloidal silicon dioxide may also be added. Finally, flavoring agents, such as peppermint, methyl salicylate, or orange flavoring may be added to the oral compositions.

Where the oral composition is in the form of a syrup, the formulation will generally consist of a suspension or solution of the compound or salt in a liquid carrier. Examples of liquid carriers are: ethanol, peanut oil, olive oil, glycerin, or water.

Where the composition is in the form of a tablet, any pharmaceutical carrier routinely used for preparing solid formulations may be used including: magnesium stearate, terra alba, talc, gelatin, acacia, stearic acid, starch, lactose, and sucrose. A tablet may be made by compression or molding, optionally with one or more accessory ingredients. Compressed tablets may be prepared by compressing in a suitable machine the active compound in a free-flowing form such as a powder or granules, optionally mixed with a binder, lubricant, inert diluent, surface active agent or dispersing agent. Molded tablets may be made by molding in a suitable machine a mixture of the powdered compound(s) moistened with an inert liquid diluent. The tablets may optionally be coated or scored and may be formulated so as to provide slow or controlled release of the active compound therein.

Where the composition is in the form of a capsule, any routine encapsulation is suitable, for example, a hard or soft gelatin capsule shell.

In controlled release formulations, biodegradable and/or biocompatible polymers may be used, such as ethylene vinyl acetate, polyanhydres, polyglycolic acid, collagen, polyorthoesters, and polyactic acid.

Typical compositions for administration by inhalation, are in the form of a solution, suspension or emulsion that may be administered as a dry powder or in the form of an aerosol using a conventional propellant such as fluroinated hydrocarbons (e.g., trichlorofluoromethane). Compositions in the form of a dry powder may contain a suitable powder base (carrier) such as lactose or starch and may be presented in different primary packaging systems (e.g., capsules and cartridges or blisters) for use in an inhaler or insufflator.

Packaging of the formulation may be suitable for unit dose or multi-dose delivery. In the case of multi-dose delivery, the formulation may be pre-metered or metered in use. Dry powder inhalers are classified into three groups: 1) single dose, 2) multiple unit dose, and 3) multi-dose devices.

For single dose inhalers, single doses may be weighed by the manufacturer into small containers, which are typically hard gelatin capsules. A capsule is taken from a separate box or container and inserted into a receptacle area of the inhaler. Next, the capsule is opened or perforated with pins or cutting blades in order to allow part of the inspiratory air stream to pass through the capsule for powder entrainment or to discharge the powder from the capsule through the perforations during inhalation. After inhalation, the emptied capsule is removed from the inhaler.

Some capsule inhalers have a magazine from which individual capsules may be transferred to a receiving chamber, in which perforation and emptying takes place. Other capsule inhalers have revolving magazines with capsule chambers that may be brought in line with the air conduit for dose discharge.

Blister inhalers provide better moisture protection of the medicament than capsule inhalers. Access to the powder is obtained by perforating the cover as well as the blister foil; or by peeling off the cover foil.

Multi-dose inhalers usually do not contain pre-measured quantities of the powder formulation. They generally consist of a container and a dose measuring principle that is operated by the patient. The container bears multiple doses that are isolated individually by volumetric displacement. Various dose measuring principles exist, including rotatable membranes or disks, rotatable cylinders, and rotatable frustums, all having cavities which have to be filled with powder from the container. Other multi dose devices have measuring slides or measuring plungers with a local or circumferential recess to displace a certain volume of powder from the container to a delivery chamber or an air conduit.

Apart from applications through dry powder inhalers, the compositions of the invention may be administered as aqueous solutions or suspensions or as aerosols delivered from pressurized packs. Aerosol compositions suitable for inhalation may be either a suspension or a solution and generally contain the active ingredient(s) and a suitable propellant such as a fluorocarbon or hydrogen-containing chlorofluorocarbon, or mixtures thereof. The aerosol compositions may be excipient free or may optionally contain additional formulation excipients (e.g., oleic acid or lecithin) and cosolvents (e.g., ethanol). Pressurized formulations will generally be retained in a canister closed with a valve and fitted into an actuator provided with a mouthpiece.

Medicaments for administration by inhalation desirably have a controlled particle size. The optimum particle size for inhalation into the bronchial system is usually 1-10 µm, preferably 2-5 um. Particles having a size above 20 µm are generally too large when inhaled to reach the small airways. To achieve these particle sizes the particles of the active compound(s) may be size reduced by conventional means, such as microization. The desired fraction may be separated out by air classification or sieving. Preferably, the particles will be crystalline.

Pressurized aerosol compositions will generally be filled into canisters fitted with a valve, especially a metering valve. Canisters may optionally be coated with a plastic material (e.g., a fluorocarbon polymer). Canisters may be fitted into an actuator adapted for buccal delivery.

Typical compositions for nasal delivery include those mentioned above for inhalation and further include non-pressurized compositions in the form of a solution or suspension in an inert vehicle such as water, optionally in combination with conventional excipients such as buffers, antimicrobials, mucoadhesive agents, tonicity modifying agents and viscosity modifying agents which may be administered by nasal pump.

Typical dermal and transdermal formulations comprise a conventional aqueous or non-aqueous vehicle. For example, the vehicle may be a cream, ointment, lotion, or paste, or may be in the form of a medicated plaster, patch or membrane.

Typical compositions for parenteral, intradermal, or subcutaneous application may include a sterile diluent such as water, saline solution, fixed oils, polyethylene glycols, glycerin, propylene glycol, or other synthetic solvents. The composition may also include antibacterial agents such as benzyl alcohol or methyl parabens, antioxidants such as ascorbic acid or sodium bisulfite, chelating agents such as ethylenediaminetetraacetic acid, and buffers such as acetates, citrates or phosphates. The composition may also include agents for the adjustment of tonicity such as sodium chloride or dextrose, and agents for the adjustment of pH including acids such as hydrochloric acid or bases such as sodium hydroxide. The composition may be enclosed in ampoules, disposable syringes or multiple dose vials made of glass or plastic.

Pharmaceutical compositions suitable for injectable use include sterile aqueous solutions or dispersions and sterile powders for the extemporaneous preparation of sterile injectable solutions or dispersion. For intravenous administration, suitable carriers include physiological saline, bacteriostatic water, or phosphate buffered saline. In all cases, the composition should be sterile and should be fluid to the extent that easy syringability exists. It should be stable under the conditions of manufacture and storage and should be preserved against the contaminating action of microorganisms such as bacteria and fungi. The carrier may be a solvent or dispersion medium, for example, water, ethanol, polyol (e.g., glycerol, propylene glycol, and liquid polyethylene glycol, etc.), and suitable mixtures thereof. The proper fluidity may be maintained, for example, by the use of a coating such as lecithin, by the maintenance of the required particle size in the case of dispersion, and by the use of surfactants. Prevention of the action of microorganisms may be achieved by various antibacterial and antifungal agents, for example, parabens, chlorobutanol, phenol, ascorbic acid, thimerosal, and the like. In many cases, the composition will include isotonic agents, for example, sugars, olyalcohols such as mannitol, sorbitol, or sodium chloride. Prolonged absorption of the injectable compositions may be brought about by including in the composition an agent which delays absorption, for example, aluminum monostearate and gelatin.

Sterile injectable solutions may be prepared by incorporating the active compound(s) in the required amount in an appropriate solvent with one or a combination of ingredients enumerated above, as required, followed by filtered sterilization. Generally, dispersions are prepared by incorporating the active compound(s) into a sterile vehicle which contains a basic dispersion medium and the required other ingredients from those enumerated above. Vacuum drying and freeze-drying, which yield a powder of the active ingredient plus any additional desired ingredient from a previously sterile-filtered solution thereof, may be used for preparing sterile powders for use in sterile injectable solutions.

The composition of the instant invention may be in unit dosage form, for example, a tablet, capsule or metered aerosol dose, so that a single dose may be administered to the patient.

The amount of each active compound which is required to achieve the desired therapeutic effect will, of course, vary with the particular compound, the route of administration, the subject under treatment, and the particular disease, disorder or condition being treated. In a combination therapy, since the individual actions of the active compounds may be mutually positively influencing and reinforcing, it may be possible to reduce the respective doses of the combined administration of the active compounds, compared with the customary doses for the individual compounds when administered alone. Thus, standard experimental testing may be performed to identify additional therapeutically effective doses.

In general, each dosage unit for oral administration may contain from about 0.3 mg to about 100 mg of the active compound of the invention. Each dosage unit for parenteral administration may contain from about 0.1 mg to about 100 mg of the active compound. Each dosage unit for intranasal administration may contain about 1 µg to about 400 µg of the active compound per activation. A dry powder inhalation dose may contain about 1 µg to about 1000 µg of the active compound per dose unit. A topical formulation contains suitably about 0.001% to about 5.0% of an active compound of the instant invention.

In an embodiment of the invention, tetomilast may be administered to a patient at about 25 mg to about 100 mg per dose. In another embodiment, tetomilast may be administered at about 25 mg to about 75 mg per dose. In yet another embodiment, tetomilast may be administered at about 50 mg per dose. In another embodiment, tetomilast may be administered orally.

A beta₂-adrenergic receptor agonist may be administered at about 25 µg to about 800 µg per dose. In an embodiment of the invention, the beta₂-adrenergic receptor agonist may be administered at about 90 µg to about 720 µg per dose. In another embodiment, the beta₂-adrenergic receptor agonist may be administered at about 720 µg over a course of 90 minutes. In yet another embodiment, the beta₂-adrenergic receptor agonist may be administered at about 90 µg per dose. The beta₂-adrenergic receptor may be administered by inhalation. In a specific embodiment, the beta₂-adrenergic receptor agonist is albuterol. In another embodiment, the beta₂-adrenergic receptor agonist is salmeterol xinafoate. Salmeterol xinafoate may be administered from about 50 µg to about 200 µg per dose. In yet another embodiment, salmeterol xinafoate may be administered at about 50 µg per dose.

An anti-inflammatory steroid may be administered at about 25 µg to 2000 µg per dose. In an embodiment of the invention, the anti-inflammatory steroid may be administered at about 50 µg to about 1000 µg per dose. In another embodiment, the anti-inflammatory steroid may be administered at about 250 µg per dose. The anti-inflammatory steroid may be administered by inhalation. In a specific embodiment, the anti-inflammatory steroid may be fluticasone propionate.

The active compounds may be administered from one to six times a day. In an embodiment of the invention, the active compounds are administered once or twice a day.

The active compounds may be administered together in individual, binary or triple dosage forms (or more), or they may be administered as different formulations. They may be administered at the same time, or they may be administered either close in time or remotely, such as where one or two compound(s) is/are administered in the morning and the other compound(s) is/are administered in the evening. Thus, for example, a beta₂-adrenergic receptor agonist and anti-inflammatory steroid of the invention may be formulated together in a binary dosage form and tetomilast as an individual dosage form, and the two dosage forms may be administered simultaneously, separately or sequentially. The combination may be used prophylactically or after the onset of symptoms has occurred. In some instances, the combination may be used to prevent the progression of a respiratory disease, disorder, or condition, or to arrest the decline of respiratory function.

In an embodiment of the invention, all active compounds may be administered at the same time, or very close in time.

The active compounds according to the invention may be administered in the form of compositions for oral administration, or inhalation delivered with the help of inhalers, especially dry powder inhalers. For example, tetomilast may be administered orally, and the beta₂-adrenergic receptor agonist and the anti-inflammatory steroid may be administered by inhalation. However, any other form or application is possible.

Examples

The description which follows is for the purpose of illustrating the instant invention and is not intended to in any way create limitations, express or implied, upon the scope of the instant invention. The claims appended hereto are for the purpose of reciting the instant invention, of expressing the contemplated scope thereof, and of pointing out particulars thereof.

Methods

The following methods were used in the studies described below.

Blood Collection and Analysis

Blood was collected for determination of tetomilast, albuterol, and fluticasone propionate concentrations. Predose baseline collections were drawn on before treatment. Pharmacokinetic (PK) blood samples were collected at predose and postdose. A total of approximately 70 mL of blood were drawn from each subject for PK analyses.

For the determination of tetomilast and fluticasone propionate concentrations, 5 mL blood samples were collected into green-top (tetomilast) or lavender-top (fluticasone propionate) Vacutainer^{®} tubes (containing heparin anticoagulant). Each tube was gently inverted 3 to 4 times and placed in an ice bath for not more than 15 minutes. Each tube was spun in a refrigerated centrifuge (4°C) for a minimum of 10 minutes at 2000-3000 rotations per minute (rpm). The separated plasma was divided equally between two polypropylene tubes and stored at -20°C or below. One tube was shipped for analysis, and the other tube was stored at -20°C or colder until the end of the study.

For the determination of albuterol concentrations, 5 mL blood samples were collected into red-top Vacutainer^{®} tubes. Each tube was allowed to clot at room temperature for 30 minutes. Each tube was spun in a refrigerated centrifuge (4°C) for a minimum of 10 minutes at 2000-3000 rpm. The separated serum was then divided equally between two polypropylene tubes and stored at -20°C or below. One tube was shipped for analysis, and the other tube was stored at -20°C or colder until the end of the study.

Pharmacodynamic Assessments

Twelve-lead electrocardiograms (ECGs) were conducted after the subject had been resting and supine for ≥ 3 minutes. In addition to screening and pretreatment baseline (Day 0) measurements, ECGs were also recorded at specific times during the treatment period. All 12-lead ECG monitors used for this study were calibrated and standardized. The ventricular rate, PR interval (the time elapsing between the beginning of the P wave and the beginning of the QRS interval in an electrocardiogram, corresponding to the electrical activity through the atria), QRS interval (the movement of electrical impulses through the ventricles), QT/QTc interval (the QT interval measures the time between the start of the Q wave and the end of the T wave in the heart's electrical cycle; QTc is a corrected QT interval, as determined by Fridericia's formula [QTcF] and Bazett's formula [QTcB]), and the overall ECG interpretation (normal or abnormal) were documented. Measurements were recorded prior to collection of any blood samples, where applicable. At least three original copies of each ECG (one each for the site, for the sponsor, and for the central ECG provider) were made. Each original ECG was signed and dated by the investigator. ECGs were reread by eResearch Technologies, Inc. (Philadelphia, PA).

Blood samples were collected for determination of serum potassium levels at screening, baseline (Day 0), and at specific times during the treatment period.

Vital signs (heart rate and systolic blood pressure) were measured after the subject had been sitting for ≥ 3 minutes. In addition to screening and baseline (Day 0), vital signs were taken at specific times during the treatment period. Measurements were recorded prior to the collection of any blood samples, where applicable.

Pulmonary Function Tests (PFTs)

Pulmonary function tests were conducted throughout the study to monitor the subjects' respiratory status. Flow volume curves were collected via spirometry. Combivent^{®} (Boehringer Ingleheim Pharmaceuticals, Inc., Ridgefield CT), which was used for short-acting rescues as needed throughout the studies described below, was withheld for 6 hours prior to any spirometric testing. Spirometry testing was performed at screening, baseline (Day 0), and at specific times during the treatment period. Each subject could have up to 5 spirometric readings at any study time point. Spirometric readings were performed using equipment from the central provider, Quantum Research, Inc. (Louisville, CO). All spirometry readings were recorded and sent to Quantum Research, Inc. for analysis

For the spirometric readings, the best effort was reported as follows. The highest acceptable forced expiratory volume in one second (FEV₁) value was selected as the "best" of the given test session. If two or more FEV₁ values were tied as highest, the sum of FEV₁ and forced vital capacity (FVC) for those efforts was used. If both conditions were equal between the two efforts, the values reported were taken from the effort that occurred first, chronologically, based on date and time of the effort.

EXAMPLE 1 : Coadministration of tetomilast and albuterol to patients with chronic obstructive pulmonary disease (COPD).

In this example, the effect of administering a combination of tetomilast and albuterol was examined.

This study was a multicenter, randomized, double-blind, placebo-controlled, crossover, multiple dose, outpatient study in 27 patients with COPD. All subjects underwent a 28-day washout from inhaled corticosteroids and/or long-acting bronchodilators and/or continuous oxygen therapy (≥16 hours per day) and a 7-day washout from oral beta₂-agonists and/or oral theophylline salts prior to baseline. Randomized subjects received an 8-day administration of oral placebo or tetomilast 50 mg oral dose (2 x 25 mg tablets) once daily (QD). On study Day 8, four consecutive doses of inhaled albuterol (90 µg albuterol base per puff) were given via a metered dose inhaler (MDI) and holding chamber (Ventolin^{®}, GlaxoSmithKline, Research Triangle Park, NC), one dose every 30 minutes over the course of 90 minutes at doses of 90 µg + 90 µg + 180 µg + 360 µg for a total dose of 720 µg (albuterol base) or 8 puffs.

Subjects entered a 7-day washout period (study days 9 through 15) and were then crossed over to the alternate treatment arm (placebo or tetomilast 50 mg oral dose). On study [0105] Day 23, the inhaled albuterol multiple dosing was repeated.

On Days 1-6 and 17-21, subjects were instructed to take their oral study medication (placebo or tetomilast 50 mg) QD with 8 ounces of water immediately before breakfast. Subjects assigned to the placebo arm received two placebo tablets QD. On Days 7, 8, 16, 22, and 23, subjects took their oral medication (placebo or tetomilast 50 mg) at the clinic in the presence of the study staff.

Combivent^{®} inhalation aerosol use was allowed for short-acting rescue as needed throughout the study except on study days where PK samples were collected, on days when the multiple doses of inhaled albuterol were administered in the clinic, and/or on days when spirometry testing was performed. On all study days where PK samples were collected, Combivent^{®} was withheld 6 hours prior to the PK draw. Additionally, on study Days 8 and 23 (when the multiple doses of inhaled albuterol were administered in the clinic), Combivent^{®} was withheld 6 hours prior to PK assessment and throughout the duration of the clinic visit. Combivent^{®} was withheld for six hours prior to spirometry testing. The recommended dose of Combivent^{®} Inhalation Aerosol is two inhalations four times a day. In this study, subjects were permitted to take additional inhalations as required; however, the total number of inhalations was not to exceed 12 in 24 hours. Subjects were to "test-spray" three times before using for the first time and in cases where the aerosol had not been used for more than 24 hours.

An albuterol reversibility challenge test was administered at screening. The albuterol reversibility challenge consisted of the following three steps: (1) subjects performed a baseline spirometry test for measurements of FEV₁; (2) subjects received two puffs of inhaled albuterol, 3 to 5 minutes apart; and (3) 30 minutes after receiving the second dose of inhaled albuterol, subjects performed a second spirometry test for measurement of FEV₁.

On Day 8 and Day 23, multiple doses of inhaled albuterol, were given at 0 hour (1 puff), 30 minutes (1 puff), 1 hour (2 puffs), and 1.5 hours (4 puffs). Pulmonary function tests and pulse oximetry were assessed predose, then every 5 and 25 minutes after each successive dose of inhaled albuterol (5, 25, 35, 55, 65, 85, 95, and 115 minutes) and at 175, 235, 295, and 355 minutes. Reversibility was not assessed at Day 8 and Day 23 as flow-volume curves were evaluated at the intervals specified above.

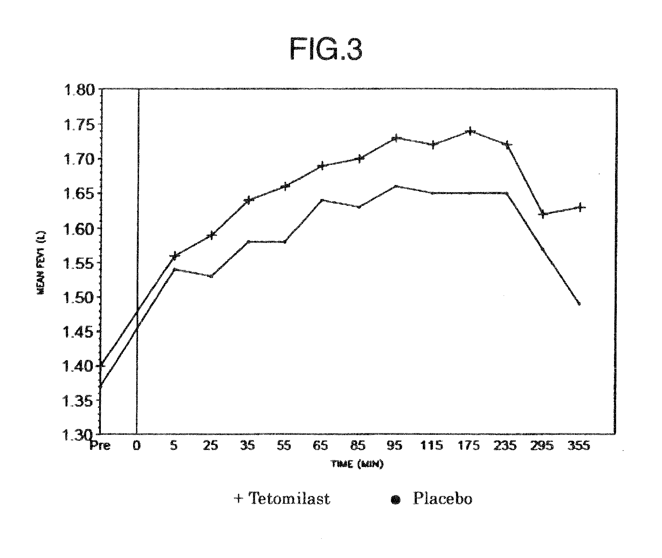

Results of pulmonary function tests revealed that the measures of patient respiratory status were consistently higher in the tetomilast group than in the placebo group. For example, the average changes from the steady-state baseline measurements to the Day 8/23 measurements of FEV₁ ranged from 0.16 to 0.34 L in the tetomilast treatment group and from 0.13 to 0.29 L in the placebo treatment group (Table 1, Figure 3). These differences were shown to be statistically significant.

| **Table 1. Mean and Change from Baseline in FEV₁** | | | | | |
|---|---|---|---|---|---|
| **Time point** | | **Steady-state (Predose) Baseline^{a}** | | **Baseline (Day 0)^{a}** | |
| | | **Tetomilast 50 mg** | **Placebo** | **Tetomilast 50 mg** | **Placebo** |
| Baseline value^{a} | | | | | |
| | Mean (SD) | 1.40(0.61) | 137(0.60) | 1.40(0.54) | 137(0.55) |
| | N | 25 | 25 | 26 | 25 |
| Day 8/23^{b} | | | | | |
| 5 min postdose^{c} | | | | | |
| | Mean (SD) | 0.16(0.09) | 0.18(0.12) | 0.19(0.19) | 0.17(0.14) |
| | N | 24 | 25 | 24 | 25 |
| 25 min postdose | | | | | |
| | Mean (SD) | 0.19(0.16) | 0.16(0.15) | 0.21(0.20) | 0-15(0-16) |
| | N | 25 | 25 | 25 | 25 |
| 35 min postdose^{c} | | | | | |
| | Mean (SD) | 0.24 (0.18) | 0.22 (0.16) | 0.26(0.18) | 0.21(0.18) |
| | N | 25 | 25 | 25 | 25 |
| 55 min postdose | | | | | |
| | Mean (SD) | 0.26 (0.20) | 0.21(0.17) | 0.28(0.19) | 0.20(0.18) |
| | N | 25 | 25 | 25 | 25 |
| 65 min postdose^{c} | | | | | |
| | Mean (SD) | 030(020) | 0-25(0.19) | 032(0.19) | 0.25 (0.18) |
| | N | 24 | 24 | 24 | 24 |
| 85 min postdose | | | | | |
| | Mean (SD) | 031(0.20) | 0.26(0.18) | 0.33(0.19) | 023(0.17) |
| | N | 25 | 25 | 25 | 25 |
| 95 min postdate^{c} | | | | | |
| | Mean (SD) | 0.34 (0.21) | 0.29 (0.20) | 0.36 (0.19) | 0.29 (0.20) |
| | N | 25 | 25 | 25 | 25 |
| 115 min postdose | | | | | |
| | Mean (SD) | 033 (0.20) | 0.28 (0.19) | 0.35 (0.18) | 6.28 (0.17) |
| | N | 25 | 25 | 25 | 25 |
| 175 min postdose | | | | | |
| | Mean (SD) | 0.34 (0.21) | 0.28 (0.23) | 036 (0.21) | 0.27 (0.21) |
| | N | 25 | 25 | 25 | 25 |
| 235 min postdose | | | | | |
| | Mean (SD) | 0.32 (0.25) | 0.28 (0.19) | 0.34 (0.25) | 0.27 (0.16) |
| | N | 25 | 25 | 25 | 25 |
| 295 min postdose | | | | | |
| | Mean (SD) | 0.2 (0.26) | 0.20 (0.25) | 0.25 (0.29) | 0.20 (0.20) |
| | N | 25 | 25 | 25 | 25 |
| 355 min postdose | | | | | |
| | Mean (SD) | 0.23 (0.22) | 0.13 (0.27) | 0.25 (0.24) | 0.12 (0.22) |
| | N | 25 | 25 | 25 | 25 |

| | | | | | |
|---|---|---|---|---|---|
| ^{a}Baselines are defined as follows: Steady-state (predose) baseline = the last nonmissing predose value obtained on Day 8 (prior to inhaled albuterol administration) in Period I or Day 23 in Period 2. Day-0 baseline = the last nonmissing predose value obtained on Day 0 in Period I or Day 16 in Period 2. ^{b}Day 8/23 = Day 8 in Period 1 or Day 23 in Period 2. ^{c}Inhaled albuterol was administered at 0 minutes (1 puff), 30 minutes (1 puff), 60 minutes (2 puffs), and 90 minutes (4 puffs). Values obtained at 5 minutes, 35 minutes, 65 minutes, and 95 minutes postdose were 5 minutes after inhaled albuterol dosing. | | | | | |

Likewise, the mean changes from baseline measurements to Day 8/23 measurements of FVC in the tetomilast group were consistently higher than those in the placebo group at each time point after the initial inhaled albuterol dosing, except for at 5 minutes postdose. The mean changes of the steady-state baseline to Day 8/23 in FVC ranged from 0.27 to 0.53 L in the tetomilast treatment group and from 0.15 to 0.43 L in the placebo treatment group (Table 2). Again, these differences were shown to be statistically significant.

| **Table 2. Mean and Change from Baseline in FVC** | | | | | |
|---|---|---|---|---|---|
| **Time point** | | **Steady-state (Predose) Baseline^{a}** | | **Baseline (Day 0)^{a}** | |
| | | **Tetomilast 50 mg** | **Placebo** | **Tetomilast 50 mg** | **Placebo** |
| Baseline value^{a} | | | | | |
| | Mean (SD) | 2.67 (0.88) | 2.67(0.83) | 2.68 (0.77) | 2.72 (0.88) |
| | N | 25 | 25 | 26 | 25 |

| Day 8/23^{b} | | | | | |
|---|---|---|---|---|---|
| 5 min postdose^{c} | | | | | |
| | Mean (SD) | 0.27 (0.25) | 029 (0.31) | 0.30 (0.28) | 0.25 (0-30) |
| | N | 24 | 25 | 24 | 25 |
| 25 min postdose | | | | | |
| | Mean (SD) | 0.29 (0.37) | 0.26 (0.28) | 0-31 (0.38) | 0.21 (0.26) |
| | N | 25 | 25 | 25 | 25 |
| 35 min postdose^{c} | | | | | |
| | Mean (SD) | 0.39 (0.36) | 0.38 (0.40) | 4.41 (0.34) | 0.33 (0.28) |
| | N | 25 | 25 | 25 | 25 |
| 55 min postdose | | | | | |
| | Mean (SD) | 0.36 (0.37) | 0.30 (0.34) | 0.38 (0.31) | 0.25 (028) |
| | N | 25 | 25 | 25 | 25 |
| 65 min postdose^{c} | | | | | |
| | Mean (SD) | 0.50 (0.40) | 0.37 (0.42) | 0.52 (0.37) | 0.33 (0.29) |
| | N | 24 | 24 | 24 | 24 |
| 85 min postdose | | | | | |
| | Mean (SD) | 0.45 (0.39) | 0.40 (0.44) | 0.47 (0.37) | 0.35 (0.30) |
| | N | 25 | 25 | 25 | 25 |
| 95 min postdose^{c} | | | | | |
| | Mean (SD) | 0.53 (0.42) | 0.41 (0.48) | 0.55 (0.40) | 0.36 (0.36) |
| | N | 25 | 25 | 25 | 25 |
| 115 min postdose | | | | | |
| | Mean (SD) | 0.51 (0.48) | 0.40 (0.36) | 0.53 (0.44) | 0.36 (0.27) |
| | N | 25 | 25 | 25 | 25 |
| 175 min postdose | | | | | |
| | Mean (SD) | 0.50 (0.38) | 0.43 (0.44) | 0.52 (0.36) | 0.39 (0.37) |
| | N | 25 | 25 | 25 | 25 |
| 235 min postdose | | | | | |
| | Mean (SD) | 0.40 (0.50) | 0.38 (0.44) | 0.42 (0.48) | 0.34 (0.35) |
| | N | 25 | 25 | 25 | 25 |
| 295 min postdose | | | | | |
| | Mean (SD) | 0.35 (0.50) | 0.24 (0.51) | 0.37 (0.49) | 0.19 (0.40) |
| | N | 25 | 25 | 25 | 25 |
| 355 min postdose | | | | | |
| | Mean (SD) | 0.32 (0.43) | 0.15 (0.51) | 0.34 (0.39) | 0.10 (0.42) |
| | N | 25 | 25 | 25 | 25 |

| | | | | | |
|---|---|---|---|---|---|
| ^{a}Baselines are defined as follows: Steady-state (predose) baseline = the last nonmissing predose value obtained on Day 8 (prior to inhaled albuterol administration) in Period 1 or Day 23 in Period 2. Day-0 baseline = the last nonmissing predose value obtained on Day 0 in Period 1 or Day 16 in Period 2. ^{b}Day 8/23 = Day 8 in Period 1 or Day 23 in Period 2. ^{c}Inhaled albuterol was administered at 0 minutes (1 puff), 30 minutes (1 puff), 60 minutes (2 puffs), and 90 minutes (4 puffs). Values obtained at 5 minutes, 35 minutes, 65 minutes, and 95 minutes postdose were 5 minutes after inhaled albuterol dosing. | | | | | |

Furthermore, although the study was not powered to detect differences in improvements in FEV₁ or FVC, exploratory repeated measures analysis showed that there is a possible potentiation of the effect of inhaled albuterol when combined with tetomilast. As evident from the larger mean FEV₁ and FVC values during the albuterol challenge on Day 8/23, there appeared to be greater efficacy and prolonged duration of action of inhaled albuterol with tetomilast compared with inhaled albuterol alone (Tables 3 and 4), Because pulmonary function test measurements were taken only up to 6 hours after inhaled albuterol administration, it is not known how long this effect would continue. These calculations provide evidence to suggest that the effect of the combined therapy is more than additive and that the effect of inhaled albuterol is potentiated when combined with tetomilast.

| **Table 3.** | **Repeated Measures ANOVA for Change From Baseline in FEV1 (L) by Time on Day 8/23 With Inhaled Albuterol (Tetomilast Verus Placebo)** | | | | | | | |
|---|---|---|---|---|---|---|---|---|
| **Time point** | **Steady-state (Predose) Baseline^{a}** | | | | **Baseline (Day 0)^{a}** | | | |
| | **Tetamilast 50 mg** | **Placebo** | **Estimated Treatment Effect** | **P-value^{b}/95% CI** | **Tetomilast 50 mg** | **Placebo** | **Estimated Treatment Effect** | **P-value^{b}/ 95% CI** |
| Baseline value^{a} | | | | | | | | |
| Mean | 1.40 | 1.37 | - | - | 1.40 | 1.37 | - | - |
| N | 25 | 25 | | | 26 | 25 | | |
| 5 min postdose^{d} | | | | | | | | |
| LS Mean^{c} | 0.16 | 0.18 | -0.01 | 0.6561 | 0.19 | 0.17 | 0.02 | 0.6427 |
| N | 24 | 25 | | - 0.07-0.04 | 24 | 25 | | -0.08-0.12 |
| 25 min postdose | | | | | | | | |
| LS mean^{c} | 0.19 | 0.16 | 0.03 | 0.3325 | 0.21 | 0.15 | 0.06 | 0.2042 |
| N | 25 | 25 | | -0.03-0.10 | 25 | 25 | | -0.03-0.15 |
| 35 min postose^{d} | | | | | | | | |
| LS Mean^{c} | 0.24 | 0.22 | 0.03 | 0.4325 | 0.26 | 0.21 | 0.05 | 0.2179 |
| N | 25 | 25 | | -0.04-0.09 | 25 | 25 | | -0.03-0.14 |
| 55 min postdose | | | | | | | | |
| LS Mean ^{c} | 0.27 | 0.21 | 0.05 | 0.0576 | 0.28 | 0.20 | 0.08 | 0.0434 |
| N | 25 | 25 | | -0.00-0.11 | 25 | 25 | | 0.00-0.16 |
| 65 min postdose^{d} | | | | | | | | |
| LS Mean^{c} | 0.30 | 0.25 | 0.06 | 0.0783 | 0.32 | 0.25 | 0.08 | 0.1234 |
| N | 24 | 24 | | -0.01-0.12 | 24 | 24 | | -0.02-0.17 |
| 85 min ρostdose | | | | | | | | |
| LS Mean^{c} | 0.31 | 0.26 | 0.05 | 0.1093 | 0.33 | 0.25 | 0.07 | 0.0970 |
| N | 25 | 25 | | -0.01-0.11 | 25 | 25 | | -0.01-0.16 |
| 95 min postdose^{d} | | | | | | | | |
| LS Mean^{c} | 0.34 | 0.29 | 0.05 | 0.1964 | 0.36 | 0.29 | 0.07 | 0.1160 |
| N | 25 | 25 | | -0.03 - 0.12 | 25 | 25 | | -0.02-0.16 |
| 115 min postdose | | | | | | | | |
| LS Mean^{c} | 033 | 0.28 | 0.05 | 0.1943 | 0.35 | 0.28 | 0.07 | 0.0922 |
| N | 25 | 25 | | -0.03 - 0.12 | 25 | 25 | | -0.01- 0.16 |
| 175 min postdose | | | | | 0.36 | | | |
| LS Mean^{c} | 0.34 | 0.28 | 0.06 | 0.1035 | | 0.27 | 0.09 | 0.0970 |
| N | 25 | 25 | | -0.01-0.14 | 25 | 25 | | -0.02-0.19 |
| 235 min postdose | | | | | | | | |
| LS Mean^{c} | 0.32 | 0.28 | 0.04 | 0.3547 | 0.34 | 0.27 | 0.07 | 0.1803 |
| N | 25 | 25 | | -0.05 -0.13 | 25 | 25 | | -0.03-0.17 |
| 295 min postdose | | | | | | | | |
| LS Mean^{c} | 0.23 | 0.20 | 0.03 | 0.5748 | 0.25 | 0.19 | 0.05 | 0.3445 |
| N | 25 | 25 | | -0.07-0.12 | 25 | 25 | | -0.06-0.17 |
| 355 min postdose | | | | | | | | |
| LS Mean^{c} | 0.23 | 0.12 | 0.11 | 0.0148 | 0.25 | 0.12 | 0.13 | 0.0167 |
| N | 25 | 25 | | 0.02-0.19 | 25 | 25 | | 0.03-0.24 |

| | | | | | | | | |
|---|---|---|---|---|---|---|---|---|
| Cl = confidence interval. ^{a}Baselines are defined as follows: Steady-state (predose) baseline= the last nonmissing predose value obtained on Day 8 (prior to inhaled albuterol administration) in Period I or Day 23 in Period 2. Day-0 baseline = the last nonmissing predose value obtained on Day 0 in Period 1 or Day 16 in Period 2. ^{b}The p-values were derived from Student t-tests on estimates of treatment comparisons (tetomilast versus placebo) which were based on LS means. ^{c}The LS means were the adjusted means from a repeated measure ANOVA with terms sequence, subject (sequence), period, and treatment. ^{d}Inhaled albuterol was administered at 0 minutes (1 puff), 30 minutes (1 puff), 60 minutes (2 puffs), and 90 minutes (4 puffs). Values obtained at 5 minutes, 35 minutes, 65 minutes, and 95 minutes postdose were 5 minutes post inhaled albuterol dosing. | | | | | | | | |

| **Table 4.** | **Repeated Measures ANOVA for Change From Baseline in FVC (L) by Time on Day 8/23 With Inhaled Albuterol (Tetomilast Versus Placebo)** | | | | | | | |
|---|---|---|---|---|---|---|---|---|
| **Time point** | **Steady-state (Predose) Baseline^{a}** | | | | **Baseline (Day 0)^{a}** | | | |
| | **Tetomilast 50 mg** | **Placebo** | **Estimated Treatment Effect** | **P-value^{b}/ 95% CI** | **Tetomilast 50 mg** | **Placebo** | **Estimated Treatment Effect** | **P-value^{b}/ 95% CI** |
| Baseline value^{a} | | | | | | | | |
| Mean | 2.67 | 2.67 | - | - | 2.68 | 2.72 | - | - |
| N | 25 | 25 | | | 26 | 25 | | |
| 5 min postdose ^{d} | | | | | | | | |
| LS Mean^{c} | 0.30 | 0.30 | 0.01 | 0.8873 | 0.31 | 0.24 | 0.06 | 0.4633 |
| N | 24 | 25 | | -0.13-0.15 | 24 | 25 | | -0.11-0.24 |
| 25 min postdose | | | | | | | | |
| LS Mean^{c} | 0.29 | 0.26 | 0.03 | 0.5436 | 0.31 | 0.21 | 0.11 | 0.1978 |
| N | 25 | 25 | | -0.08-0.14 | 25 | 25 | | -0.06-0.28 |
| 35 min postdose^{d} | | | | | | | | |
| LS Mean^{c} | 0.39 | 0.38 | 0.01 | 0.8619 | 0.41 | 0.33 | 0.09 | 0.2613 |
| N | 25 | 25 | | -0.11-0.13 | 25 | 23 | | -0.07-0.24 |
| 55 min postdose | | | | | | | | |
| LS Mean^{c} | 0.37 | 0.30 | 0.07 | 0.2965 | 0.39 | 0.24 | 0.15 | 0.0691 |
| N | 25 | 25 | | -0.07-0.20 | 25 | 25 | | -0.01-0.30 |
| 65 min postdose ^{d} | | | | | | | | |
| LS Mean ^{c} | 0.50 | 0.37 | 0.13 | 0.1139 | 0.51 | 0.33 | 0.18 | 0.0661 |
| N | 24 | 24 | | -0.03-0.30 | 24 | 24 | | -0.01-0.38 |
| 85 min postdose | | | | | | | | |
| LS Mean ^{c} | 0.45 | 0.40 | 0.05 | 0.5013 | 0.48 | 0.35 | 0.13 | 0.1531 |
| N | 25 | 25 | | -0.10-0.20 | 25 | 25 | | -0.05-0.30 |
| 95 min postdose^{d} | | | | | | | | |
| LS Mean^{c} | 0.53 | 0.41 | 0.12 | 0.2350 | 0.55 | 0.36 | 0.19 | 0.0852 |
| N | 25 | 25 | | -0.08-0.32 | 25 | 25 | | -0.03-0.42 |
| 115 min postdose | | | | | | | | |
| LS Mean^{c} | 0.51 | 0.41 | 0.11 | 0.2694 | 0.54 | 0.35 | 0.18 | 0.1009 |
| N | 25 | 25 | | -0.09-0.30 | 25 | 25 | | -0.04.0.40 |
| 175 min postdose | | | | | | | | |
| LS Mean^{c} | 0.50 | 0.43 | 0.07 | 0.2669 | 0.53 | 0.38 | 0.15 | 0.1524 |
| N | 25 | 25 | | -4.06-0.21 | 25 | 25 | | -0.06-036 |
| 235 min postdose | | | | | | | | |
| LS Mean^{c} | 0.39 | 0.38 | 0.01 | 0.9256 | 0.42 | 0.33 | 0.09 | 0.4991 |
| N | 25 | 25 | | -0.21-0.23 | 25 | 25 | | -0.17-0.34 |
| 295 min postdose | | | | | | | | |
| LS Mean^{c} | 0.35 | 0.24 | 0.11 | 0.2363 | 0.38 | 0.19 | 0.19 | 0.1252 |
| N | 25 | 25 | | -0.08-0.31 | 25 | 25 | | -0.06-0.44 |
| 355 min postdose | | | | | | | | |
| LS Mean^{c} | 0.32 | 0.15 | 0.17 | 0,0230 | 0.34 | 0.09 | 0.24 | 0.0134 |
| N | 25 | 25 | | 0.03-0.31 | 25 | 25 | | 0.06-0.43 |

| | | | | | | | | |
|---|---|---|---|---|---|---|---|---|
| Cl = confidence interval. ^{a}Baselines are defined as follows: Steady-state (predose) baseline = the last nonmissing predose value obtained on Day 8 (prior to inhaled albuterol administration) in Period 1 or Day 23 in Period 2. Day-0 baseline = the last nonmissing predose value obtained on Day 0 in Period 1 or Day 16 in Period 2. ^{b}The p-values were derived from Student t-tests on estimates of treatment comparisons (tetomilast versus placebo) which were based on LS means. ^{c}The LS means were the adjusted means from a repeated measure ANOVA with terms sequence, subject (sequence), period, and treatment. ^{d}Inhaled albuterol was administered at 0 minutes (1 puff), 30 minutes (1 puff), 60 minutes (2 puffs), and 90 minutes (4 puffs). Values obtained at 5 minutes, 35 minutes, 65 minutes, and 95 minutes postdose were 5 minutes after inhaled albuterol dosing. | | | | | | | | |

No clinically meaningful changes were observed between oral tetomilast and placebo, both used concomitantly and with relatively high doses of inhaled albuterol, in heart rate, systolic blood pressure, serum potassium, or QT interval. Furthermore, no clinically meaningful differences were observed between oral tetomilast and placebo, both used concomitantly with inhaled albuterol, in adverse events, serum chemistry, hematology, or urinalysis. These results demonstrate the safety of the combined therapy comprising tetomilast and albuterol in patients.

Taken together, the results of this study demonstrate that the combination of tetomilast and albuterol is a safe and effective therapy for enhancing pulmonary function in patients with COPD. Furthermore, the results suggest that when administered as part of a combined therapy, the compounds are more efficacious than when administered alone. Thus, the combination of tetomilast and albuterol presents a promising treatment for respiratory diseases, disorders, or conditions.

EXAMPLE 2: Coadministration of tetomilast, salmeterol xinafoate, and fluticasone propionate to patients with COPD.

In this example, the effect of administering a combination of tetomilast, salmeterol xinafoate, and fluticasone propionate was examined.

This study was a multicenter, randomized, double-blind, placebo-controlled, parallel group, outpatient, phase 1B study of Advair Diskus^{®}250/50 (fluticasone propionate and salmeterol xinafoate, GlaxoSmithKline, Research Triangle Park, NC) administered concomitantly with oral placebo or 50 mg tetomilast (2 x 25 mg tablets) in patients with COPD. All patients underwent a minimum 28-day washout from short-acting inhaled corticosteroids, long-acting bronchodialators, and/or continuous oxygen therapy (≥16 hours per day), and a minimum 7-day washout from beta₂-agonists and/or oral theophylline. Patients were randomly assigned to one of the two treatment arms (oral tetomilast 50 mg or placebo) prior to dosing on Day 1, and were distributed 1:1 between the two groups according to a computer-generated randomization code provided by the Otsuka Maryland Research Institute (OMRI) Biostatistics Department. Randomization was performed using a central IVRS system and was centralized across study centers.

Subjects received 7 days of treatment with tetomilast 50 mg or placebo oral dose in treatment period 1. During treatment period 2, Days 8 through 35, concomitant inhaled Advair Diskus^{®} 250/50 was added to the treatment regimen.

Subjects were instructed to take their oral study medication (tetomilast 50 mg or placebo) QD with 8 ounces of water immediately after breakfast for all study treatment days. Subjects assigned to the placebo group received two placebo tablets QD. Subjects assigned to the tetomilast group received 2 x 25 mg tetomilast tablets QD. On study Days 1-7, 14, 21, 28, and 35, subjects consumed their oral study medication (tetomilast 50 mg or placebo) at the clinic in the presence of the study staff.

The inhaled Advair Diskus^{®} 250/50 was self-administered by subjects on all study days between Days 8 and 35, except for Days 14, 21, 28, and 35, when the medication was administered at the clinic in the presence of the study staff. Advair Diskus^{®} 250/50 was administered twice daily (BID). The morning dose coincided with the timing of the oral dose (tetomilast 50 mg or placebo). The evening dose was taken 12 hours later. Dosing times were recorded in subject diaries.

Combivent^{®} (Boehringer Ingleheim Pharmaceuticals, Inc., Ridgefield, CT) was provided by the sponsor for short-acting rescue use during the study. Combivent^{®} was withheld for 6 hours prior to any PFTs. Combivent^{®} was supplied as a metered-dose inhaler. Each actuation meters 21 µg of ipratropium bromide and 120 µg of albuterol sulfate from the valve and delivers 18 mg of ipratropium bromide and 103 µg of albuterol sulfate (equivalent to 90 µg albuterol base) from the mouthpiece. The dose of Combivent^{®} was two inhalations four times a day. In this study, subjects were permitted to take additional inhalations as required; however, the total number of inhalations was not to exceed 12 in 24 hours. It was recommended to "test-spray" three times before using the inhaler for the first time and in cases where the aerosol had not been used for more than 24 hours.

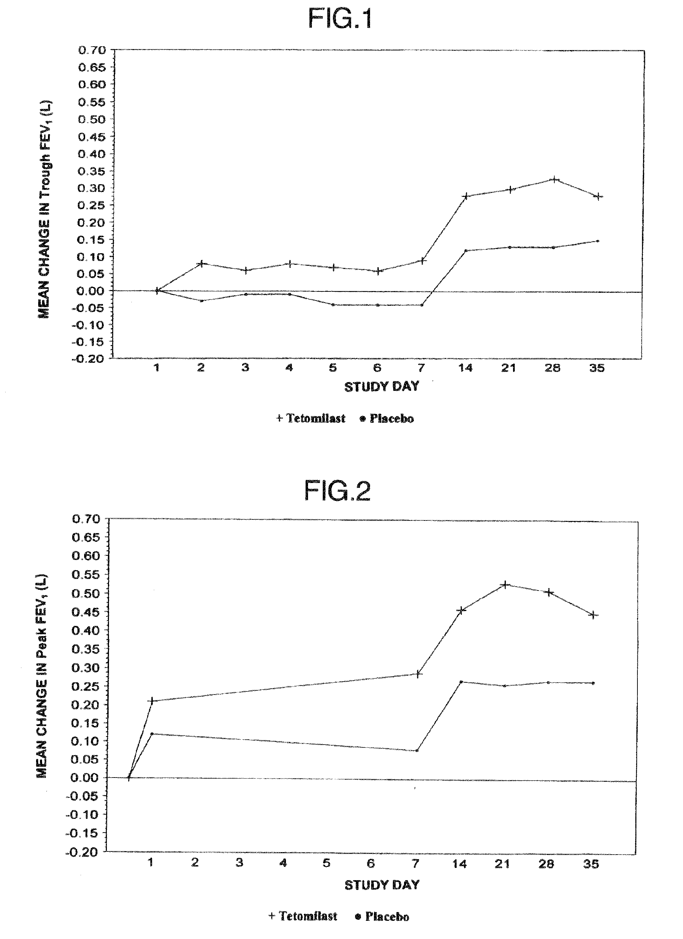

Results of pulmonary function tests revealed that the measures of patient respiratory status were consistently higher in the tetomilast group than in the placebo group. For example, using the day-1 baseline, FEV₁ levels were statistically significantly improved in the tetomilast group compared with placebo group. Trough FEV₁ was statistically significantly improved in the tetomilast group compared with placebo at all time points through day-7 (prior to coadministration with Advair Diskus^{®} 250/50), except for at day-3, and at all time points from day-14 through day-35 (after coadministration of Advair Diskus^{®} 250/50) (Figure 1). For peak and average FEV₁, the improvements in the tetomilast group were statistically significant compared with placebo at days 7, 21, and day 28 and approached statistical significance at day 14 and day 35 (Figure 2).

In addition, although the study wasn't powered to detect differences in improvements in FEV₁, exploratory repeated measures analysis showed that there is a possible potentiation of the effect of inhaled fluticasone propionate plus salmeterol (Advair Diskus^{®} 250/50) when combined with tetomilast. As evident from the larger mean FEV₁ values during the period of Advair Diskus^{®} 250/50 coadministration on days 8 through 35, there appeared to be greater efficacy and prolonged duration of action of inhaled Advair Diskus^{®} 250/50 with tetomilast compared with inhaled Advair Diskus^{®} 250/50 alone (Tables 5 and 6). These calculations provide compelling evidence to suggest that the effect of the combined therapy is more than additive and that the effect of inhaled salmeterol xinafoate and fluticasone propionate is potentiated when combined with tetomilast. Furthermore, this study supports and extends the results from EXAMPLE 1, which examined the effect of tetomilast plus cumulative doses of albuterol.

| **Table 5**. **ANCOVA in Change From Baseline in Trough FEV₁ (Tetomilast Versus Placebo)** | | | | | | | | |
|---|---|---|---|---|---|---|---|---|
| **Visit** | **Baseline Day 1 Predose**^{a} | | | | **Baseline Day 7 Predose^{a}** | | | |
| | **Tetomilast 50 mg/ Advair Diskus^{b} LS Mean^{c}** | **Placebo/ Advair Diskus**^{b} **LS Mean**^{c} | **Estimated Treatment Effect^{d}** | **P-value^{e}/ 95% CI** | **Tetomilast 50 mg/ Advair Diskus^{b} LS Mean^{c}** | **Placebo/ Advair Diskus**^{b} **LS Mean^{c}** | **Estimated Treatment Effect^{d}** | **P-value^{e}/ 95% Cl** |
| **Baseline Mean** | 1.25 | 1.02 | 0.22 | 0.1224 | 1.38 | 1.00 | 0.38 | 0.0174 |
| | n = 22 | n = 22 | | -0.06-0.51 | n = 20 | n = 21 | | 0.07-0.69 |
| Day 2 | 0.08 | -0.03 | 0.11 | 0.0086 | -- | -- | -- | -- |
| | n = 21 | n = 22 | | 0.03-0.20 | | | | |
| Day 3 | 0.06 | -0.01 | 0.07 | 0.1129 | -- | -- | -- | -- |
| | n = 20 | n = 21 | | -0.02-0.16 | | | | |
| Day 4 | 0.09 | -0.03 | 0.12 | 0.0038 | -- | -- | -- | -- |
| | n = 20 | n = 21 | | 0.04-0.20 0.20 | | | | |
| Day 5 | 0.08 | -0.04 | 0.13 | 0.0066 | -- | -- | -- | -- |
| | n=20 | n = 21 | | 0.04-0.21 | | | | |
| Day 6 | 0.07 | -0.05 | 0.11 | 0.0271 | -- | -- | -- | -- |
| | n = 20 | n = 21 | | 0.01-0.21 | | | | |
| Day 7 | 0.09 | -0.04 | 0.13 | 0.0113 | -- | -- | -- | -- |
| | n = 20 | n = 21 | | 0.03-0.23 | | | | |
| Day 14 | 0.28 | 0.12 | 0.16 | 0.0381 | 0.18 | 0.16 | 0.02 | 0.7031 |
| | n = 19 | n = 21 | | 0.01-0.31 | n = 19 | n = 21 | | -0.09 - 0.14 |
| Day 21 | 0.30 | 0.13 | 0.18 | 0.0245 | 0.21 | 0.6 | 0.05 | 0.4245 |
| | n = 19 | n = 21 | | 0.02-0.34 | n = 19 | n = 21 | | -0.07-0.16 |
| Day 28 | 0.34 | 0.13 | 0.21 | 0.0371 | 0.24 | 0.18 | 0.06 | 0.4021 |
| | n = 19 | n = 21 | | 0.01-0.41 | n = 19 | n = 21 | | -0-08-0.20 |
| Dray 35 | 0.29 | 21 0.14 | 0.15 | 0.0451 | 0.20 | 0.18 | 0.02 | 0.6976 |
| | n = 19 | n = 21 | | 0.00-0.30 | n = 19 | n = 21 | | -0.10-0-14 |

| | | | | | | | | |
|---|---|---|---|---|---|---|---|---|
| CI = confidence interval. ^{a}Baselines are defined as follows: Baseline Day 1 predose = the last nonmissing predose value on Day 1. Baseline Day 7 predose = the last nonmissing predose value on Day 7. ^{b}Subjects in the tetomilast group received oral tetomilast 50 mg QD on Days 1 through 7 and oral tetomilast 50 mg QD+ Advair Diskus 250/50 BID on Days 8 through 35. Subjects in the placebo group received oral placebo tablets QD on Days 1 through 7 and oral placebo tablets + Advair Diskus 250/50 on Days 8 through 35. ^{c}The LS means were the adjusted means from an ANCOVA model with terms for treatment as the main effect and baseline as covariate. ^{d}The estimated treatment effect is the difference between the two LS means. ^{e}The p-values were derived from Student t-tests on estimates of treatment comparisons (tetomilast versus placebo) which were based on LS means. | | | | | | | | |

| | **Table 6. ANCOVA in Change From Baseline (Day 1 Predose) in Peak FEV1 (Tetomilast versus Placebo)** | | | |
|---|---|---|---|---|
| **Visit** | **Change From Baseline in Peak FEV₁** | | | |
| | **Tetomilast 50 mg/ Advair Diskus^{b} LS Mean ^{c}** | **Placebo/ Advair Diskus^{b} LS Mean^{c}** | **Estimated Treatment Effect^{d}** | **P-value^{e}/ 95% CI** |
| **Baseline Mean** | 1.25 | 1.02 | 0.22 | 0.1224 |
| | n = 22 | n = 22 | | -0.06-0.51 |
| Day 1 | 0.20 | 0.12 | 0.08 | 0.3569 |
| | n = 22 | n = 22 | | -0.10-0.26 |
| Day 7 | 0.30 | 0.07 | 0.23 | 0.0408 |
| | n = 20 | n = 21 | | 0.01-0.44 |
| Day 14 | 0.47 | 0.26 | 0.21 | 0.0841 |
| | n = 19 | n = 21 | | -0.03-0.45 |
| Day 21 | 0.55 | 0.25 | 0.30 | 0.0386 |
| | n = 19 | n = 21 | | 0,02-0.58 |
| Day 28 | 0.54 | 0.25 | 0.28 | 0.0198 |
| | n = 19 | n = 21 | | 0.05-0.52 |
| Day 35 | 0.46 | 0.26 | 0.21 | 0.0751 |
| | n = 19 | n = 21 | | -0.02-0.44 |

| | | | | |
|---|---|---|---|---|
| CI = confidence interval. ^{a}-Baseline Day 1 predose = the last nonmissing predose value on Day 1. Baseline Day 7 predose = the last nonmissing predose value on Day 7. ^{b}Subjects in the tetomilast group received oral tetomilast 50 mg QD on Days 1 through and oral tetomilast 50 mg QD + Advair Diskus 250/50 BID on Days 8 through 35. Subjects in the placebo group received oral placebo tablets QD on Days 1 through 7 and oral placebo tablets + Advair Diskus 250/50 on Days 8 through 35. ^{c}The LS means were the adjusted means from an ANCOVA model with terms for treatment as the main effect and baseline as covariate. ^{d}The estimated treatment effect is the difference between the two LS means. ^{e}The p-values were derived from Student t-tests on estimates of treatment comparisons (tetomilast versus placebo) which were based on LS means. | | | | |

No clinically meaningful changes were observed between oral tetomilast and placebo, both used concomitantly and in combination with Advair Diskus^{®} 250/50, in heart rate, systolic blood pressure, serum potassium, or QT interval. Furthermore, no clinically meaningful differences were observed between oral tetomilast and placebo, both used concomitantly with inhaled Advair Diskus^{®} 250/50, in adverse events, serum chemistry, hematology, or urinalysis. These results demonstrate the safety of the combined therapy comprising tetomilast, salmeterol xinafoate, and fluticasone propionate in patients.

Taken together, the results of this study demonstrate that the combination of tetomilast, salmeterol xinafoate, and fluticasone propionate is a safe and effective therapy for increasing bronchodilatory response in patients with COPD. Furthermore, the results suggest that when administered as part of a combined therapy, the compounds are more efficacious than when administered alone. Thus, the combination of tetomilast, salmeterol and fluticasone presents a promising treatment for respiratory diseases, disorders, or conditions.

Other embodiments of the invention will be apparent to those skilled in the art from consideration of the specification and practice of the invention disclosed herein. It is intended that the specification and examples be considered as exemplary only, with a true scope and spirit of the invention being indicated by the following claims.

### Industrial Applicability

The instant invention may be used to treat or prevent a respiratory disease, disorder, or condition, such as chronic obstructive pulmonary disease (COPD).

## Claims

1. A combination of tetomilast and at least one beta₂-adrenergic receptor agonist for use in the prevention or treatment of a respiratory disease, disorder or condition.

2. The combination according to claim 1, wherein the beta₂-adrenergic receptor agonist is selected from albuterol (salbutamol), AR-C68397AA, arformoterol, bambuterol, bitolterol, broxaterol, carbuterol, CHF-1035, clenbuterol, dopexamine, fenoterol, formoterol, hexoprenaline, HOKU-81, ibuterol, isoetharine, isoprenaline, KUL-1248, levosalbutamol, mabuterol, meluadrine, metaproterenol, nolomirole, orciprenaline, pirbuterol, procaterol, reproterol, rimiterol, ritodrine, salmefamol, salmeterol, sibenadet, soterenot, sulfonterol, TA-2005, terbutaline, tiaramide, tulobuterol, epinephrine, norepinephrine, colterol, ethyinorepinephrine, isoproterenol, metaproterenol, ephedrine, GSK-597901, GSK-159797, GSK-678007, GSK-642444, GSK-159802, (-)-2-[7(S)-[2(R)-Hydroxy-2-(4-hydroxyphenyl)ethylamino]-5, 6,7,8-terahydro-2-naphthyloxy]-N,N-dimethylacetamide hydrochloride monohydrate, carmoterol, QAB-149 and 5-[2-(5,6-diethylindan-2-ylamino)-1-hydroxyethyl]-8-hydroxy-1H-quinolin-2-one, 4-hydroxy-7-[2-{[2-{[3-(2-phenylethoxy)propyl]sulfonyl} ethyl]amino} ethyl]-2(3H)-benzothiazolone, 1-(2-fluoro-4-hydroxyphenyl)-2-[4-(1-benzimidazolyl)-2-methyl-2-butylamino]ethanol, 1-[3-(4-methoxybenzylamino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1- [2H-5-hydroxy-3-oxo-4H-l, 4-benzoxazin-8-yl] -2- [3- (4-n-butyloxyphenyl)-2-methyl-2-propylamino]ethanol, 1-[2H-5-hydroxy-3-oxo-4H-l,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-one, 1-(4-amino-3-chloro-5-trifluoromethylphenyl)-2-tertbutylamino)ethanol, or 1-(4-ethoxycarbonylamino-3-cyano-5-fluorophenyl)-2-(tert-butylamino)ethanol.

3. The combination according to claim 2, wherein the beta₂-adrenergic receptor agonist is salmeterol, salmeterol xinafoate, albuterol, or a stereoisomer of albuterol.

4. The combination according to claim 1, wherein the beta₂-adrenergic receptor agonist is a long-acting beta₂-adrenergic receptor agonist.

5. The combination according to claim 1, wherein the beta₂-adrenergic receptor agonist is a short-acting beta₂-adrenergic receptor agonist.

6. The combination according to claim 1, wherein the tetomilast and beta₂-adrenergic receptor agonist are administered simultaneously, separately, or sequentially.

7. The combination according to claim 1, wherein the tetomilast is administered orally.

8. The combination according to claim 1, wherein the beta₂-adrenergic receptor agonist is administered by inhalation.

9. The combination according to any of the claims 1-8, wherein administering amount of tetomilast to the patient is from 25 mg to 100 mg per dose.

10. The combination according to any of the claims 1-9, wherein administering amount of beta₂-adrenergic receptor agonist to the patient is from 25 µg to 800 µg per dose.

11. The combination according to any of the claims 1-10, further comprising at least one anti-inflammatory steroid.

12. The combination according to claim 11, wherein the anti-inflammatory steroid is selected from alcolmetasone dipropionate, beclomethasone, budesonide, butixocort propionate, ciclesonide, clocortolone pivalate, deflazacort, dexamethasone, dexamethasone palmitoate, dexamethasone sodium phosphate, deprodone propionate, fimexolone, fluocinolone acetonide, fluocinonide, flunisolide, fluticasone, fluticasone propionate, halobetasol propionate, halopredone acetate, halometasone, hydrocortisone, hydrocortisone aceponate, hydrocortisone acetate, hydrocortisone sodium succinate, hydrocortisone probutate, loteprednol etabonate, methylprednisolone, methylprednisolone aceponate, methylprednisolone suleptanate, mometasone, naflocort, prednisone, prednisolone, prednisolone farnesylate, prednisolone sodium phosphate, prednicarbate, rimexolone, rofleponide, triamcinolone, tipredane, 6α,9β-difluoro-17α-[(2-furanylcarbonyl)oxy]-11β-hydroxy-16α-methyl-3-oxo-androsta-1,4-diene-17b-carbothioic acid S-fluoromethyl ester, 6α,9β-difluoro-11β-hydroxy-16α-methyl-3-oco-17β-propionyloxy-androsta-1,4-diene-17p-carbothioic acid S-(2-oxo-tetrahydro-furan-3S-yl)ester, RPR-106541, or 5T-126 (SSP-Torii).

13. The combination according to claim 12, wherein the anti-inflammatory steroid is fluticasone or fluticasone propionate.

14. The combination according to any of the claims 11-13, wherein the tetomilast, the beta₂-adrenergic receptor agonist, and the anti-inflammatory steroid are administered simultaneously, separately, or sequentially.

15. The combination according to any of the claims 11-14, wherein the anti-inflammatory steroid is administered by inhalation.

16. The combination according to any of the claims 11-15, wherein administering amount of anti-inflammatory steroid to the patient is from 25 µg to 2000 µg per dose.

17. The combination according to any of the claims 1-16, wherein the respiratory disease, disorder or condition is selected from an asthmatic condition, acute respiratory distress syndrome, chronic or acute bronchoconstriction, chronic obstructive pulmonary disease (COPD), bronchitis, emphysema, pneumoconiosis, small airway obstruction, sinusitis, bronchiectasis or rhinitis.

18. The combination according to claim 17, wherein the respiratory disease is chronic obstructive pulmonary disease (COPD).

19. The combination according to any of the claims 11-18, wherein the patient has a steady-state plasma level of tetomilast.

20. A pharmaceutical composition comprising tetomilast and at least one beta₂-adrenergic receptor agonist.

21. The pharmaceutical composition of claim 20, wherein the beta₂-adrenergic receptor agonist is selected from salmeterol or albuterol.

22. The pharmaceutical composition of claim 20, comprising about 25 mg to about 100 mg of tetomilast and about 25 µg to about 800 µg of the beta₂-adrenergic receptor agonist.

23. The pharmaceutical composition of claim 20, further comprising at least one anti-inflammatory steroid.

24. The pharmaceutical composition of claim 23, wherein the beta₂-adrenergic receptor agonist is selected from salmeterol or albuterol and wherein the anti-inflammatory steroid is fluticasone.

25. The pharmaceutical composition of claim 24, comprising about 25 mg to about 100 mg of tetomilast, about 25 µg to about 800 µg of the beta₂-adrenergic receptor agonist, and about 25 µg to about 2000 µg of the anti-inflammatory steroid.
